# EUROPEAN PATENT SPECIFICATION

(11) **EP 4 161 589 B1**
(45) Date of publication and mention of the grant of the patent: **25.03.2026**
(21) Application number: 21740193.4
(22) Date of filing: 31.05.2021
(51) Int. Cl.: A61L 2/10, A61L 2/20, G07C 1/00

(54) **USE OF AN OPENABLE MONEY-HOLDING CONTAINER FOR THE DISINFECTION OF COINS, TOKENS, BANKNOTES, CHEQUES, SHOPPING VOUCHERS AND/OR MEAL VOUCHERS**
VERWENDUNG EINES ZU ÖFFNENDEREN GELDBEHÄLTERS ZUR DESINFEKTION VON MÜNZEN, TOKEN, BANKNOTEN, SCHECKS, EINKAUFSGUTSCHEINE UND/ODER ESSENSGUTSCHEINE
UTILISATION D'UN CONTENANT DE STOCKAGE D'ARGENT POUVANT ÊTRE OUVERT EN VUE DE LA DÉSINFECTION DE PIÈCES DE MONNAIE, DE JETONS, DE BILLETS DE BANQUE, DE CHÈQUES, BONS D'ACHAT ET/OU BONS REPAS

(30) Priority: 04.06.2020 IT 202000003115 U; 04.06.2020 IT 202000003121 U; 02.10.2020 IT 202000005566 U
(43) Date of publication of application: 12.04.2023
(73) Proprietor: SERPILLI, Roberto, 60033 Chiaravalle (AN) (IT)
(72) Inventor: SERPILLI, Roberto, 60033 Chiaravalle (AN) (IT)
(74) Representative: Biondini, Daniele
(86) International application number: PCT/IB2021/054737
(87) International publication number: WO 2021/245523

(56) References cited:
- GB-A- 2 375 425
- KR-A- 20140 081 230
- RO-A0- 130 742
- US-A1- 2011 253 563

## Description

The present invention relates to the use of an openable money-holding container for the disinfection of coins, tokens, banknotes, cheques, shopping vouchers, meal vouchers.

The field of application of the invention proposed here is that of change-giver drawers, also called money-holding drawers, and in general, that of containers for storing money in various forms.

Change-giver drawers, or money-holding drawers, are normally of two types. A first type of money-holding drawer, also called "fixed money-holding drawer", is mainly, but not exclusively, used in supermarkets and shops and has a base wherein a compartment is defined and to which a lid is connected at the top in a horizontally sliding manner or rotatable manner around a horizontal rotation axis.

The "fixed money-holding drawer" also includes a basket arranged in a fixed or removable way in the compartment of the aforesaid base.

A plurality of compartments, or housings, are obtained in the basket for storing coins, tokens, banknotes, cheques, vouchers and/or other paper securities.

A second type of money-holding drawer called "mobile money-holding drawer" or more precisely "sliding money-holding drawer", used almost everywhere, is a drawer, without a lid, which slides into and out of a seat that can be of two types: the most common seat is called "puller drawer" or, in a shortened form, "drawer" and is generally under the cash register, to which it is mechanically fixed or which is held in place by the weight of the cash register; the other type of seat is instead constituted by a special compartment or puller drawer (which for the sake of brevity is also named "drawer") integral with the cash register.

In this case, too, a compartment is defined in the drawer in which a basket is arranged, in a fixed or removable way, wherein a plurality of compartments, or housings, are obtained for storing coins, tokens, banknotes, cheques, vouchers and/or other paper securities.

A problem that is becoming increasingly felt by people today is having to handle money potentially contaminated by viruses, bacteria or other pathogens.

The technical solutions currently available on the market, in terms of fixed or mobile money-holding drawers, do not in any way solve the problem mentioned above KR 2014 0081230 A, GB 2 375 425 A, RO 130 742 A0, US 2011/253563 Al disclose money containers with disinfection systems.

The main object of the present invention is therefore to provide an openable money-holding container for storing coins, tokens, banknotes, cheques, shopping vouchers, meal vouchers, which allows the risk associated with handling potentially contaminated money to be considerably reduced, for example for customers or shop owners or other people who might use this container.

A further object of the present invention is to provide an openable money-holding container for storing coins, tokens, banknotes, cheques, shopping vouchers, meal vouchers, which allows effective disinfection of coins, banknotes and/or other securities in any material contained therein.

Another object of the present invention is to provide an openable money-holding container for storing coins, tokens, banknotes, cheques, shopping vouchers, meal vouchers, which is safe for the health of their users.

Therefore, the specific object of the invention proposed herein is the use of an openable money-holding container according to claim 1.

Further distinctive technical aspects of the present invention are provided in the dependent claims.

Characteristics and advantages will become more evident from the following description of preferred but not exclusive embodiments illustrated, purely by way of non-limiting example, in the accompanying drawings, wherein:
- figure 1 is an axonometric view of a first openable money-holding container assembly according to a first embodiment of the present disclosure
- figure 2 is an axonometric view of a second openable money-holding container assembly (in particular, a basket) according to a first embodiment of the present disclosure
- figure 3 is an exploded view of the second assembly shown in figure 2;
- figure 4 is an axonometric view of an openable money-holding container according to the above-mentioned first embodiment of the present disclosure, in an open configuration;
- figure 5 is an axonometric view of the openable money-holding container shown in figure 4, in a closed configuration;
- figure 6 is a detail view taken from figure 4;
- figure 7 is an axonometric view of a first openable money-holding container assembly according to a second embodiment of the present disclosure;
- figure 8 is an axonometric view of a second openable money-holding container assembly (in particular, a basket) according to a second embodiment of the present disclosure
- figure 9 is an axonometric view of the second assembly shown in figure 8, with some coins and banknotes stored inside it;
- figure 10 is an exploded view of the second assembly shown in figure 8;
- figure 11 is an axonometric view of an openable money-holding container according to the above-mentioned second embodiment of the present disclosure in an open configuration;
- figure 12 is an axonometric view of the openable money-holding container shown in figure 11, in a closed configuration;
- figure 13 is a first detail view taken from figure 11; and
- figure 14 is a second detail view taken from figure 11.

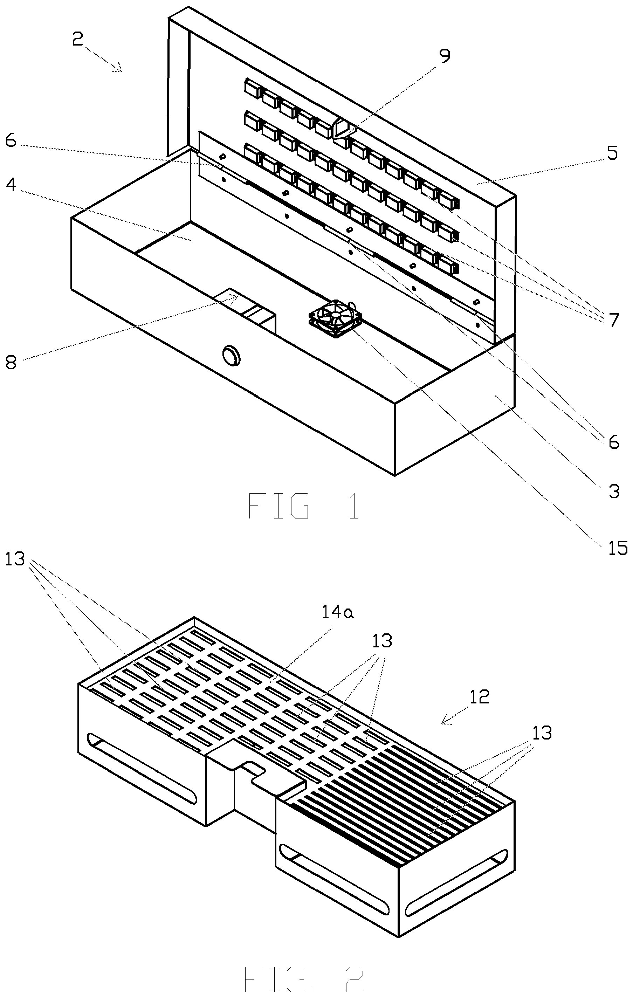

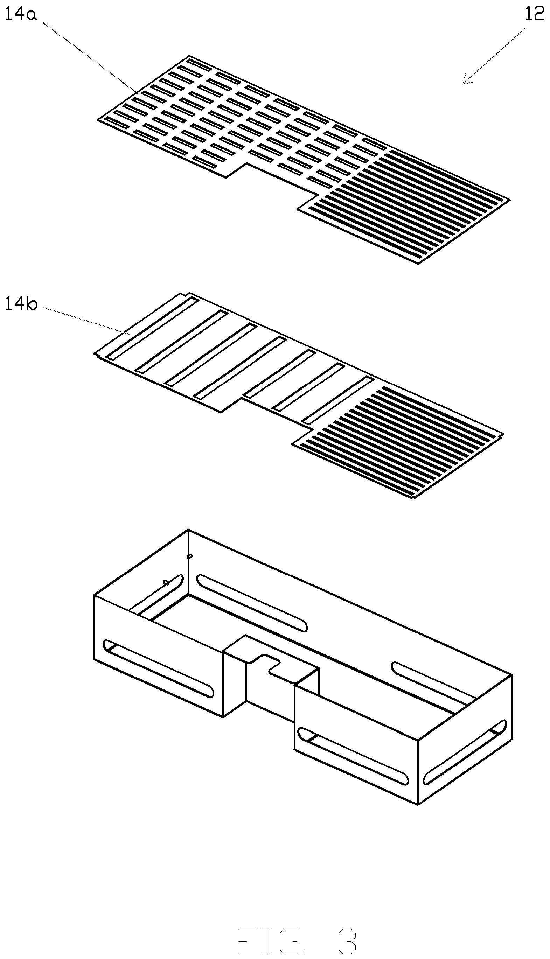

With reference to figures 1-6, 1 indicates an openable money-holding container, according to a first embodiment of the present disclosure for the disinfection of coins, tokens, banknotes, cheques, shopping vouchers, meal vouchers.

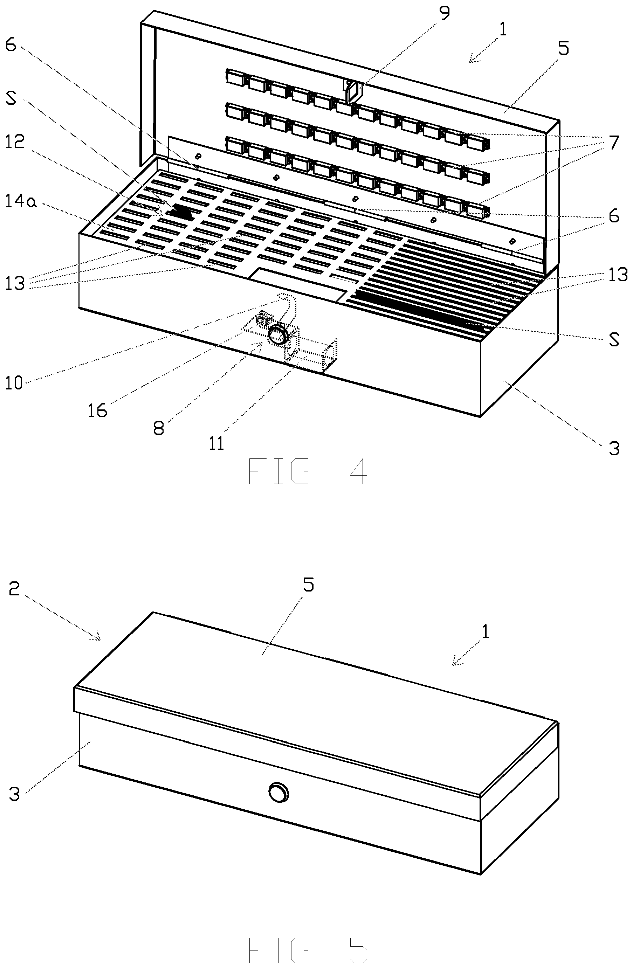

Said openable money-holding container 1 comprises a box 2, or an openable casing, formed by a hollow base 3 with a substantially parallelepiped shape, in which a compartment 4 is defined, and by a lid 5 hinged by means of a plurality of hinges 6 to said base 3 in a movable way between a closed position (see fig. 5), in which the box 2 is closed, and a position of maximum opening (see figs. 1 and 4) in which the same box 2 is completely open and the compartment 4 is totally exposed to the outside, i.e. accessible from the outside.

According to further variants of the present disclosure contrary to what is shown in figures 1-6, the aforementioned lid could be connected to its respective base in a sliding way or in another way.

On the inner face of the lid 5, i.e. on the face thereof facing the compartment 4 when the lid 5 is closed, there is a plurality of UVC ray emitters 7 (for example UVC LEDs) and, optionally, also UVA and/or UVB ray emitters to irradiate the compartment 4 and its contents when the aforementioned lid 5 is in the closed position.

The aforementioned emitters can be fixed to the lid 5 mechanically (e.g. with screws and/or joints and/or rivets, and/or clips, and/or velcro fasteners, etc.) and/or with adhesive materials (e.g. glues and/or double-sided adhesive tapes and/or adhesive surfaces, etc.) and/or by melting of own material and/or filler material.

Power supply for the aforementioned emitters is obtained from the standard power grid that powers the electrical appliances in the installation environment (with voltage possibly down-rated to lower values according to the applicable electrical safety standards) or through batteries of any type or through any other power supply; whether power supply is alternating current or direct current is immaterial since whatever the type of ultraviolet emission, there are devices capable of transforming alternating current into direct electrical current and vice versa.

Preferably, all the (upper, lower and side) walls of the box 2 are made of or internally coated with a material shielding from UV rays, e.g a metal sheet.

On the inner side of the front wall of the base 3, i.e. the opposite wall to that where the hinges 6 are mounted, there is a closing device 8 configured to work jointly with an eyelet 9 mounted on the corresponding side of the lid 5, i.e. on the side of the latter opposite to the one in which the same hinges 6 are mounted.

In particular, the closing device 8 comprises a hook 10 swinging between an engagement position, wherein it is hooked to the eyelet 9 when the lid 5 is closed, and a disengagement position, wherein the hook 10 is disengaged from the eyelet 9 when the lid 5 is closed.

The aforementioned closing device 8 also comprises a spring (not shown for greater clarity of representation), which constantly acts on the hook 10 so as to keep it in its engagement position, and a coil 11, i.e. a linear electric actuator, operating in a such a way as to move, on receiving a corresponding electrical impulse, the hook 10 to its respective disengagement position, overcoming the elastic force of the aforementioned spring and thus allowing the opening of the lid 5.

Alternatively or in addition to the electric opening system described above, a manual opening system by means of a key and lock can be provided; in this case, it will be the key itself that moves the aforementioned hook 10 so as to obtain the opening the lid 5.

The aforementioned openable money-holding container 1 also includes a basket 12 or a support wherein a plurality of seats 13 is defined for arranging coins and/or tokens and/or banknotes and/or cheques and/or shopping vouchers and/or meal vouchers.

The basket 12 may in turn comprise plates 14a, 14b which are perforated and/or slotted, spaced out and overlapping each other so as to form the aforementioned seats 13.

The basket 12 has an overall shape substantially complementary to the compartment 4 of the base 3 of the box 2, in such a way that it is possible to house, in a removable way, the basket 12 in the aforementioned compartment 4 and close the lid 5 leaving it completely inside the box. 2.

Furthermore, the basket 12 itself is preferably made of material that is transparent or substantially transparent to ultraviolet (UV) radiations, to allow the ultraviolet rays emitted by the aforementioned UVC ray emitters 7 (and possibly also by the UVA and UVB ray emitters) to go through the walls of the basket 12 and thus fully hit the banknotes and/or coins and/or other securities S placed in the seats 13.

Some examples of materials suitable for basket 12 construction are quartz glasses (which are sufficiently transparent to UVC rays), PDMS-based plastics, i.e. Polydimethylsiloxane (which are substantially transparent to UVA and UVB rays and also sufficiently transparent to UVC rays) and fluorinated ethylene propylene (FEP).

When the basket 12 with its respective coins and/or banknotes and/or other securities S is in the compartment 4 of the base 3 with the lid 5 of the box 2 in the closing position, the ultraviolet rays emitted by the UVC ray emitters 7 and by any additional emitters of UVA and/or UVB rays produce as an effect the disinfection of the basket 12 and also of the coins and/or banknotes and/or other securities S present in the related seats 13, reducing the quantity of bacteria and/or viruses and/or spores and/or moulds and/or other pathogens possibly present therein.

To effectively irradiate a greater number of money items (i.e. banknotes, vouchers, cheques, coins, tokens, cards, etc.), the seats 13 of the basket 12 according to the invention are formed by support elements (such as for example slots and/or rods and/or grooves and/or partitions and/or combs and/or grids, etc.) configured in such a way as to keep the money in vertical positions, separated from each other, ensuring that the ultraviolet rays emitted by the aforementioned emitters can reach a sufficient number of surfaces of a significant number of banknotes and/or coins and/or other securities arranged in the seats 13.

To increase the density and diffusion of ultraviolet radiations inside the openable money-holding container 1, it is possible to provide that the internal surfaces of the base 3 and/or of the lid 5, in whole or in part, reflect ultraviolet radiations.

To further increase the effectiveness of the openable money-holding container 1 in terms of disinfection of coins and/or tokens and/or banknotes and/or cheques and/or shopping vouchers and/or meal vouchers it is possible to install inside the box 2 (see attached figures) and/or in the basket 12 one or more fans 15 or natural draft systems in order to ensure the circulation of air already irradiated by the aforementioned UVC ray emitters 7 and by any additional UVA and/or UVB ray emitters inside the openable money-holding container 1.

Preferably, the aforementioned one or more fans 15 are mounted on the internal part of the bottom of the base 3 (see figure 1).

This particular technical measure allows the achievement of a further weakening and/or decrease in the quantity of bacteria and/or viruses and/or spores and/or moulds and/or other pathogens possibly present in the basket 12 (when placed in the box 2) and/or either on the money and/or inside the box 2. Furthermore, according to a variant of the openable money-holding container 1 described above, a through opening can be provided in the basket 12 which, when this basket 12 is arranged in the compartment 4, is located above the aforementioned one or more fans 15 mounted on the inside of the bottom of the base 3; this allows the passage of air through said through opening, further helping the circulation of the irradiated air inside the openable money-holding container 1.

To reduce the risk of potential deformation and overheating of the openable money-holding container 1, ventilation slots may be provided in the box 2.

To prevent ultraviolet radiation leaking out of the box 2, e.g. from any ventilation slots, when the aforementioned UVC ray emitters 7 and any additional UVA and/or UVB ray emitters are active, in the junction or connection areas of the box 2 it is possible to provide for the presence of optical shielding elements, such as for example gaskets and/or joints and/or labyrinth seals and/or extensions of the walls of the box 2 and/or extra closing panels and/or other elements or optical shielding systems.

To increase the safety of use of the openable money-holding container 1, it is also possible to install a safety device 16 in it, operatively connected with the UVC ray emitters 7 and any additional UVA and/or UVB ray emitters and also with the closing device 8, in such a way that the deactivation of the aforementioned emitters is automatically arranged when the same closing device 8 controls the opening of the lid 5 from the related closed position, and the reactivation of the same emitters only when the aforesaid closing device 8 closes the lid 5.

The safety device 16 therefore operates in such a way as to prevent the aforementioned emitters from remaining active when the lid 5 is open. According to further variants of the present disclosure the safety device 16 can be connected, rather than to the closing device 8, to one or more sensors provided in the openable money-holding container 1 and operating so as to detect when the lid 5 is completely closed and when opening of the latter is controlled from its respective closing position.

Position sensors suitable for this purpose could be, for example, electromechanical or electromagnetic microswitches, or proximity sensors, or optoelectronic and/or photoelectric infrared or laser sensors.

Alternatively or in addition to the safety device 16, a control device can be provided (not shown in the attached figures) operatively connected to the UVC ray emitters 7 and to any additional UVA and/or UVB ray emitters, which allows the user of the openable money-holding container 1 to voluntarily control both the activation and the deactivation of these emitters, so that the same user can deactivate (for example manually by means of a specific button) these emitters before opening the lid 5 and activate them following the closure of the latter.

In particular, if the aforementioned control device and the safety device 16 are both present in the openable money-holding container 1, it is possible to provide for operation whereby the deactivation of the aforementioned emitters can be voluntarily implemented by the user through the control device and their activation can be carried out automatically by the safety device 16 or voluntarily by the user by means of the same control device when the lid 5 is in its respective closed position.

The safety device 16 and the other systems provided in the openable money-holding container 1 to increase safety make it possible to avoid the risk of ultraviolet radiation leaking out of the box 2 which, in case of leakage, could cause serious damage to the users of the openable money-holding container 1, in particular to their eyes and skin.

According to further variants of the present disclosure one or more ozone lamps can be provided in place of or in addition to the aforesaid UVC ray emitters 7.

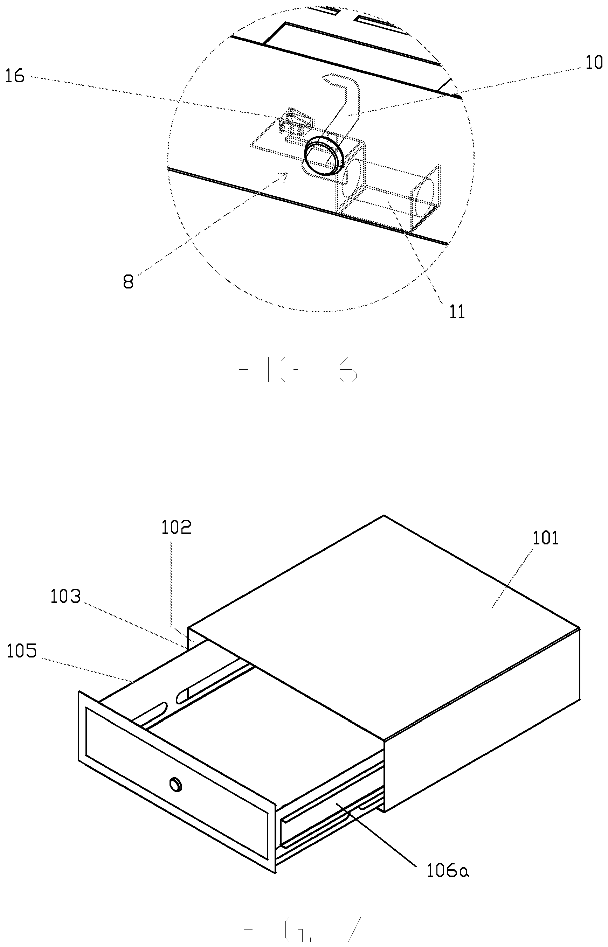

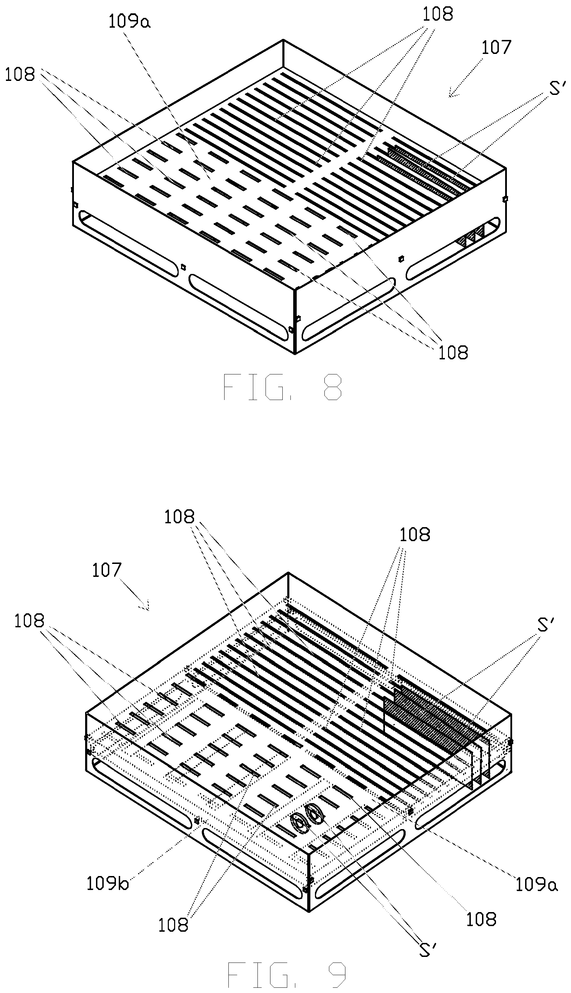

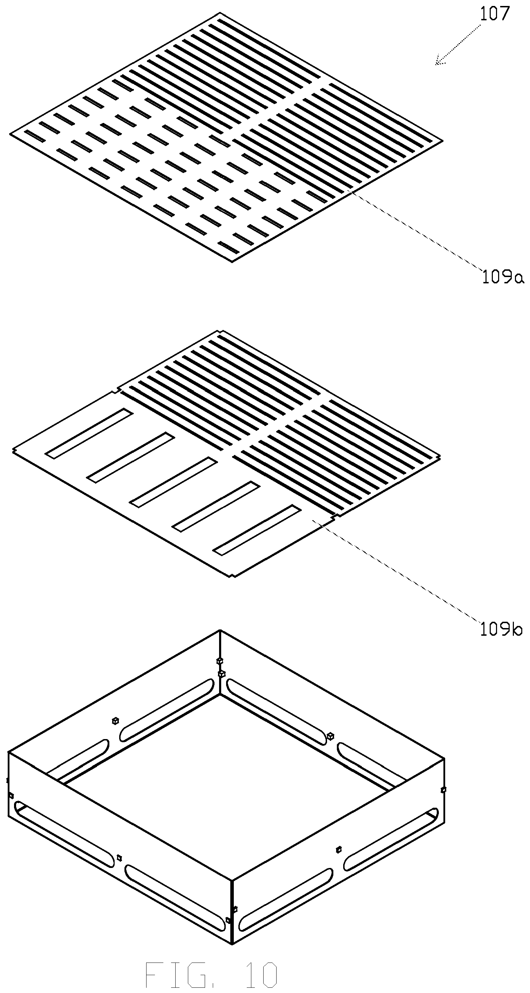

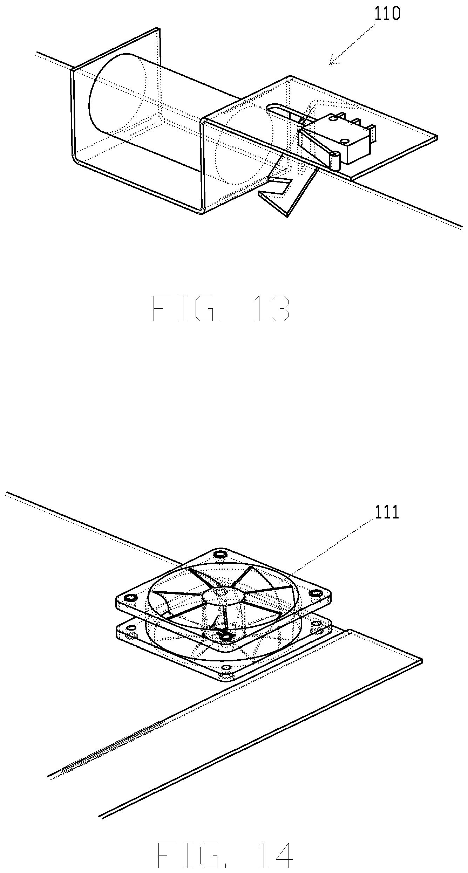

Referring now, instead, to figures 7-14, 100 indicates a second openable money-holding container for the disinfection of coins, tokens, banknotes, cheques, shopping vouchers, meal vouchers, in accordance with a second embodiment of the present disclosure.

Specifically, the second openable money-holding container 100 comprises a shell or an outer casing 101 having, preferably but not limited to, the shape of a parallelepiped.

A compartment 102 open to the outside through a front opening 103 is defined in the outer casing 101.

On the inner face of the upper wall (i.e. of the lid) of the outer casing 101 is a plurality of UVC ray emitters 104 (e.g. UVC LEDs) and, optionally, also emitters of UVA and/or UVB rays.

As regards the fixing and power supply of the UVC emitters 104 and any UVA and/or UVB emitters, the same considerations made above in relation to the openable money-holding container 1 apply.

The second openable money-holding container 100 also includes a drawer 105 configured so as to be inserted into the compartment 102 of the outer casing 101.

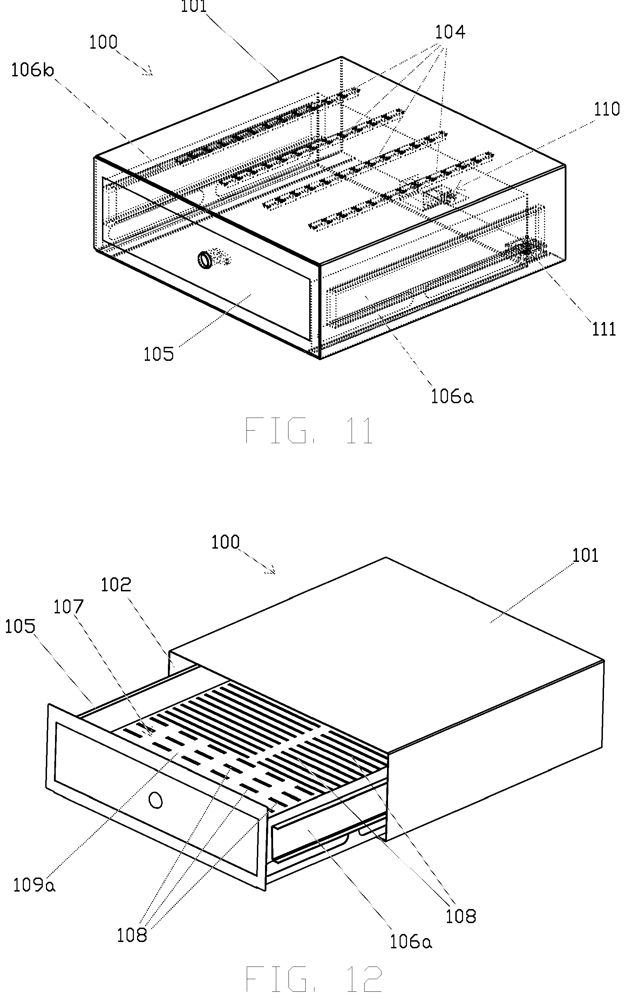

In particular, the drawer 105 is engaged in the outer casing 101 by means of two lateral guides 106a, 106b which allow it to slide between a pulled out or open position, wherein the internal part of the drawer 105 is fully or almost completely exposed to the outside namely accessible from the outside (see figs. 7 and 12), and a retracted or closed position, wherein the drawer 105 is fully inserted into the compartment 102 of the outer casing 101 (see fig. 11).

The second openable money-holding container 100 also comprises a basket 107 or a support wherein a plurality of seats 108 is defined for arranging coins and/or tokens and/or banknotes and/or cheques and/or shopping vouchers and/or meal vouchers S'.

The basket 107 can be formed internally and integrally in the drawer 105 or as a separate object that can be removably inserted inside the drawer 105, so that the money arranged in the basket 107 can be hit by the ultraviolet rays emitted by the aforementioned emitters when the drawer 105 is in its respective closed position.

As regards the conformation of the seats 108 and the materials of which the basket 107 can be composed, the same considerations made above in relation to the openable money-holding container 1 apply.

Furthermore, in this case, too, the basket 107 may comprise plates 109a, 109b which are perforated and/or slotted, spaced out and overlapping each other so as to form the aforementioned seats 108.

Preferably, all the walls of the outer casing 101 and of the drawer 105 are made of or internally coated with a material shielding against ultraviolet (UV) rays, e.g a metal sheet.

Also in the second openable money-holding container 100 a safety device 110 can be provided, operatively connected with the UVC ray emitters 104 and any additional UVA and/or UVB ray emitters, and comprising at least one sensor for detecting when the drawer 105 is completely closed and when the opening of the latter is arranged from its respective closed position.

Specifically, the safety device 110 operates in such a way as to automatically stop the emission of ultraviolet rays by the aforementioned emitters instantly when the aforementioned sensor detects the opening of the drawer 105 from its closed position, and allow the automatic reactivation of the same emitters only when the sensor detects that the drawer 105 is completely closed.

Also in this case, the reactivation of the emitters can be arranged voluntarily by the user, for example manually, instead of automatically.

The safety device 110 therefore operates in such a way as to prevent the above-mentioned emitters from remaining active when the drawer 105 is opened.

For the opening of the drawer 105, the same electric opening system described above can be provided with a coil and hook and, in addition or alternatively, the same manual opening system indicated above with a key, lock and hook.

Similarly to what is indicated above for the openable money-holding container 1, one or more fans 111 or natural draft systems can be installed in the drawer 105 and/or inside the outer casing 101 in order to ensure the circulation of air already irradiated by the aforesaid UVC ray emitters 104 and any additional UVA and/or UVB ray emitters inside the second openable money-holding container 100, thus allowing a more effective disinfection of the money present in the basket 107.

Preferably, the aforementioned one or more fans 111 are mounted on the internal side of the rear wall (i.e. the wall opposite the opening 103) of the outer casing 101 or on the internal part of the bottom of the outer casing 101, at the rear of the latter; this allows circulation of the irradiated air to be further facilitated inside the second openable money-holding container 100.

According to further variants of the present invention, one or more ozone lamps can be provided in place of or in addition to the UVC ray emitters 104.

As can be inferred from the above description, both the openable money-holding container 1 and the second openable money-holding container 100, according to the present invention, allow effective disinfection of the money contained therein to be obtained by reducing the virus and/or bacteria and/or spores and/or moulds and/or other pathogens load possibly present in said money, maintaining in all circumstances an adequate level of safety for their respective users.

## Claims

1. Use of an openable money-holding container (1; 100) with coins and/or tokens and/or banknotes and/or cheques and/or shopping vouchers and/or meal vouchers, for the disinfection of the coins and/or tokens and/or banknotes and/or cheques and/or shopping vouchers and/or meal vouchers, wherein the openable money-holding container (1; 100) comprises:
- a basket (12; 107) wherein a plurality of seats (13, 108) is defined for arranging coins and/or tokens and/or banknotes and/or cheques and/or shopping vouchers and/or meal vouchers, the seats being formed by support elements configured in such a way as to keep the coins and/or tokens and/or banknotes and/or cheques and/ or shopping vouchers and/or meal vouchers in vertical positions, separated from each other;
- a fixed part (3; 101) and a mobile part (5; 105), wherein the plurality of seats (13; 108) is provided in said fixed part (3; 101) or in said mobile part (5; 105), said mobile part (5; 105) being movable with respect to said fixed part (3; 101) between a closing position, wherein the openable money-holding container (1; 100) is closed and the plurality of seats (13; 108) is completely enclosed by the openable money-holding container (1; 100), and an opening position, wherein the openable money-holding container (1; 100) is open and the plurality of seats (13; 108) is exposed to the outside of the openable money-holding container (1; 100) namely accessible from the outside of the openable money-holding container (1; 100);
- at least one UVC ray emitter (7; 104), or at least one ozone lamp, connected to said fixed part (3; 101) or to said mobile part (5; 105) and arranged in a position such as to emit, in use, at least one beam towards the coins and/or tokens and/or banknotes and/or cheques and/or shopping vouchers and/or meal vouchers arranged in the plurality of seats (13; 108) when said mobile part (5; 105) is in said closing position, such as to reduce or cancel the charge of viruses and/or bacteria and/or spores and/or moulds and/or others pathogens possibly present in said coins and/or said tokens and/or said banknotes and/or said cheques and/or said shopping vouchers and/or said meal vouchers; and
- at least one air circulation device (15; 111) to circulate air inside the openable money-holding container (1; 100);
wherein the use is **characterized by** vertically arranging, separated from each other, the coins and/or tokens and/or banknotes and/or cheques and/or shopping vouchers and/or meal vouchers in the seats (13; 108).

2. Use of the openable money-holding container (1; 100) according to claim 1, **characterised in that** said at least one UVC ray emitter (7; 104), or said at least one ozone lamp, is arranged above the plurality of seats (13; 108) when said mobile part (5; 105) is in the closing position.

3. Use of the openable money-holding container (1) according to any one of the preceding claims, **characterised in that** said fixed part (3) has a hollow part (4) configured to house the plurality of seats (13).

4. Use of the openable money-holding container (1) according to claim 3, **characterised in that** said mobile part (5) is rotatably connected to said fixed part (3).

5. Use of the openable money-holding container (1) according to claim 3 or 4, **characterised in that** said at least one UVC ray emitter (7), or said at least one ozone lamp, is connected to said mobile part (5).

6. Use of the openable money-holding container (1) according to any one of claims 3 to 5, **characterised in that** said at least one air circulation device (15) is connected to said fixed part (3) at a bottom portion of the hollow part (4).

7. Use of the openable money-holding container (100) according to claim 1 or 2, **characterised in that** said mobile part (105) has a hollow part configured to house the plurality of seats (108).

8. Use of the openable money-holding container (100) according to claim 7, **characterised in that** said mobile part (105) is connected to said fixed part (101) in a sliding manner.

9. Use of the openable money-holding container (100) according to claim 7 or 8, **characterised in that** said at least one UVC ray emitter (104), or said at least one ozone lamp, is connected to said fixed part (101).

10. Use of the openable money-holding container (100) according to any one of claims 7 to 9, **characterised**
**in that** in said fixed part (101) a compartment (102) for housing said mobile part (105) and an opening (103) communicating with the compartment (102) are defined; and
**in that** said at least one air circulation device (111) is connected to said fixed part (101) at an area opposite to said opening (103).

11. Use of the openable money-holding container (1; 100) according to any one of the preceding claims, **characterised in that** the plurality of seats (13; 108) is removable.

12. Use of the openable money-holding container (1; 100) according to any one of the preceding claims, **characterised in that** at least one seat of said plurality of seats (13; 108) is made of a material which is at least partially transparent to said at least one ray.

13. Use of the openable money-holding container (1; 100) according to any one of the preceding claims, **characterised in that** it comprises a safety device (16; 110) operatively connected to said at least one UVC ray emitter (104), or to said at least one ozone lamp, and configured in such a way as to automatically deactivate said at least one UVC ray emitter (104) or said at least one ozone lamp when said mobile part (5; 105) moves from the closing position to the opening position.

14. Use of the openable money-holding container (1; 100) according to any one of the preceding claims, **characterised in that** it comprises a control element to allow a person to voluntarily control the deactivation of said at least one UVC ray emitter (104) or said at least one ozone lamp.

15. Use of the openable money-holding container (1; 100) according to any one of the preceding claims, **characterised in that** it comprises at least one surface which reflects said at least one ray and is defined on one side of said mobile part (5; 105) or of said fixed part (3; 101) facing towards the plurality of seats (13; 108) when said mobile part (5; 105) is in the closing position.

16. Use of the openable money-holding container (1; 100) according to any one of the preceding claims, **characterised in that** at least one seat of said plurality of seats (13; 108) is made of quartz glass or of Polydimethylsiloxane (PDMS) or of fluorinated ethylene propylene (FEP).

## Patentansprüche

1. Verwendung eines öffenbaren Geldbehälters (1; 100), der zur Aufbewahrung von Münzen und/oder Wertmarken und/oder Banknoten und/oder Schecks und/oder Einkaufsgutscheinen und/oder Essensgutscheinen dient, für die Desinfektion der Münzen und/oder Wertmarken und/oder Banknoten und/oder Schecks und/oder Einkaufsgutscheine und/oder Essensgutscheine, wobei der öffenbare Geldbehälter (1; 100) Folgendes umfasst:
- einen Einsatz (12; 107), in dem eine Vielzahl von Aufnahmen (13, 108) zum Anordnen von Münzen und/oder Wertmarken und/oder Banknoten und/oder Schecks und/oder Einkaufsgutscheinen und/oder Essensgutscheinen definiert ist, wobei die Aufnahmen durch Stützelemente gebildet werden, die dafür ausgelegt sind, die Münzen und/oder Wertmarken und/oder Banknoten und/oder Schecks und/oder Einkaufsgutscheine und/oder Essensgutscheine in senkrechten Stellungen voneinander getrennt zu halten;
- einen feststehenden Teil (3; 101) und einen beweglichen Teil (5; 105), wobei die Vielzahl von Aufnahmen (13; 108) in dem feststehenden Teil (3; 101) oder in dem beweglichen Teil (5; 105) vorgesehen ist, wobei der bewegliche Teil (5; 105) in Bezug auf den feststehenden Teil (3; 101) bewegbar ist zwischen einer Schließstellung, in welcher der öffenbare Geldbehälter (1; 100) geschlossen ist und die Vielzahl von Aufnahmen (13; 108) von dem öffenbaren Geldbehälter (1; 100) vollständig umschlossen ist, und einer Öffnungsstellung, in welcher der öffenbare Geldbehälter (1; 100) geöffnet ist und die Vielzahl von Aufnahmen (13; 108) zur Außenseite des öffenbaren Geldbehälters (1; 100) hin freiliegt, das heißt von der Außenseite des öffenbaren Geldbehälters (1; 100) aus zugänglich ist;
- zumindest einen UVC-Strahler (7; 104) oder zumindest eine Ozonlampe, der/die mit dem feststehenden Teil (3; 101) oder dem beweglichen Teil (5; 105) verbunden und in einer solchen Position angeordnet ist, dass er/sie im Gebrauch zumindest einen Strahl in Richtung der Münzen und/oder Wertmarken und/oder Banknoten und/oder Schecks und/oder Einkaufsgutscheine und/oder Essensgutscheine aussendet, die in der Vielzahl von Aufnahmen (13; 108) angeordnet sind, wenn sich der bewegliche Teil (5; 105) in der Schließstellung befindet, um die Belastung durch Viren und/oder Bakterien und/oder Sporen und/oder Schimmelpilze und/oder andere Krankheitserreger, die möglicherweise auf den Münzen und/oder Wertmarken und/oder Banknoten und/oder Schecks und/oder Einkaufsgutscheinen und/oder Essensgutscheinen vorhanden sind, zu verringern oder zu beseitigen; und
- zumindest eine Luftumwälzvorrichtung (15; 111) zum Umwälzen von Luft innerhalb des öffenbaren Geldbehälters (1; 100);
wobei die Verwendung **dadurch gekennzeichnet ist, dass** die Münzen und/oder Wertmarken und/oder Banknoten und/oder Schecks und/oder Einkaufsgutscheine und/oder Essensgutscheine in den Aufnahmen (13; 108) voneinander getrennt in senkrechter Stellung angeordnet sind.

2. Verwendung des öffenbaren Geldbehälters (1; 100) nach Anspruch 1, **dadurch gekennzeichnet, dass** der zumindest eine UVC-Strahler (7; 104), oder die zumindest eine Ozonlampe oberhalb der Vielzahl von Aufnahmen (13; 108) angeordnet ist, wenn sich der bewegliche Teil (5; 105) in der Schließstellung befindet.

3. Verwendung des öffenbaren Geldbehälters (1) nach einem beliebigen der vorhergehenden Ansprüche, **dadurch gekennzeichnet, dass** der feststehende Teil (3) einen hohlen Teil (4) aufweist, der dafür ausgelegt ist, die Vielzahl von Aufnahmen (13) aufzunehmen.

4. Verwendung des öffenbaren Geldbehälters (1) nach Anspruch 3, **dadurch gekennzeichnet, dass** der bewegliche Teil (5) drehbar mit dem feststehenden Teil (3) verbunden ist.

5. Verwendung des öffenbaren Geldbehälters (1) nach Anspruch 3 oder 4, **dadurch gekennzeichnet, dass** der zumindest eine UVC-Strahler (7), oder die zumindest eine Ozonlampe, mit dem beweglichen Teil (5) verbunden ist.

6. Verwendung des öffenbaren Geldbehälters (1) nach einem der Ansprüche von 3 bis 5, **dadurch gekennzeichnet, dass** die zumindest eine Luftumwälzvorrichtung (15) mit dem feststehenden Teil (3) an einem unteren Abschnitt des hohlen Teils (4) verbunden ist.

7. Verwendung des öffenbaren Geldbehälters (100) nach Anspruch 1 oder 2, **dadurch gekennzeichnet, dass** der bewegliche Teil (105) einen hohlen Teil aufweist, der dafür ausgelegt ist, die Vielzahl von Aufnahmen (108) aufzunehmen.

8. Verwendung des öffenbaren Geldbehälters (100) nach Anspruch 7, **dadurch gekennzeichnet, dass** der bewegliche Teil (105) gleitbar mit dem feststehenden Teil (101) verbunden ist.

9. Verwendung des öffenbaren Geldbehälters (100) nach Anspruch 7 oder 8, **dadurch gekennzeichnet, dass** der zumindest eine UVC-Strahler (104), oder die zumindest eine Ozonlampe, mit dem feststehenden Teil (101) verbunden ist.

10. Verwendung des öffenbaren Geldbehälters (100) nach einem der Ansprüche von 7 bis 9, **dadurch gekennzeichnet,**
**dass** in dem feststehenden Teil (101) ein Fach (102) zur Aufnahme des beweglichen Teils (105) und eine Öffnung (103), die mit dem Fach (102) in Verbindung steht, definiert sind; und
**dass** die zumindest eine Luftumwälzvorrichtung (111) mit dem feststehenden Teil (101) an einem der Öffnung (103) gegenüberliegenden Bereich verbunden ist.

11. Verwendung des öffenbaren Geldbehälters (1; 100) nach einem beliebigen der vorhergehenden Ansprüche, **dadurch gekennzeichnet, dass** die Vielzahl von Aufnahmen (13; 108) abnehmbar ist.

12. Verwendung des öffenbaren Geldbehälters (1; 100) nach einem beliebigen der vorhergehenden Ansprüche, **dadurch gekennzeichnet, dass** zumindest eine Aufnahme der Vielzahl von Aufnahmen (13; 108) aus einem Material hergestellt ist, das zumindest teilweise für den zumindest einen Strahl durchlässig ist.

13. Verwendung des öffenbaren Geldbehälters (1; 100) nach einem beliebigen der vorhergehenden Ansprüche, **dadurch gekennzeichnet, dass** sie eine Sicherheitsvorrichtung (16; 110) umfasst, die mit dem zumindest einen UVC-Strahler (104), oder der zumindest einen Ozonlampe, wirkverbunden und dafür ausgelegt ist, den zumindest einen UVC-Strahler (104) oder die zumindest eine Ozonlampe automatisch zu deaktivieren, wenn sich der bewegliche Teil (5; 105) aus der Schließstellung in die Öffnungsstellung bewegt.

14. Verwendung des öffenbaren Geldbehälters (1; 100) nach einem beliebigen der vorhergehenden Ansprüche, **dadurch gekennzeichnet, dass** sie ein Steuerelement umfasst, das es einer Person ermöglicht, die Deaktivierung des zumindest einen UVC-Strahlers (104) oder der zumindest einen Ozonlampe willentlich zu steuern.

15. Verwendung des öffenbaren Geldbehälters (1; 100) nach einem beliebigen der vorhergehenden Ansprüche, **dadurch gekennzeichnet, dass** sie zumindest eine Oberfläche umfasst, die den zumindest einen Strahl reflektiert und auf einer Seite des beweglichen Teils (5; 105) oder des feststehenden Teils (3; 101) so definiert ist, dass sie der Vielzahl von Aufnahmen (13; 108) zugewandt ist, wenn sich der bewegliche Teil (5; 105) in der Schließstellung befindet.

16. Verwendung des öffenbaren Geldbehälters (1; 100) nach einem beliebigen der vorhergehenden Ansprüche, **dadurch gekennzeichnet, dass** zumindest eine Aufnahme der Vielzahl von Aufnahmen (13; 108) aus Quarzglas oder aus Polydimethylsiloxan (PDMS) oder aus fluoriertem Ethylenpropylen (FEP) hergestellt ist.

## Revendications

1. Utilisation d'un récipient porte-argent ouvrable (1; 100) contenant des pièces de monnaie et/ou jetons et/ou billets de banque et/ou chèques et/ou bons d'achat et/ou chèques-repas, pour la désinfection des pièces de monnaie et/ou jetons et/ou billets de banque et/ou chèques et/ou bons d'achat et/ou chèques-repas, ledit récipient porte-argent ouvrable (1; 100) comprend:
- un panier (12; 107) dans lequel une pluralité de sièges (13, 108) est définie pour disposer des pièces de monnaie et/ou jetons et/ou billets de banque et/ou chèques et/ou bons d'achat et/ou chèques-repas, les sièges étant constitués d'éléments de support configurés de manière à maintenir les pièces de monnaie et/ou jetons et/ou billets de banque et/ou chèques et/ou bons d'achat et/ou chèques-repas dans des positions verticales, séparés les uns des autres;
- une partie fixe (3; 101) et une partie mobile (5; 105), où la pluralité de sièges (13; 108) est prévue dans ladite partie fixe (3; 101) ou dans ladite partie mobile (5; 105), ladite partie mobile (5; 105) étant mobile par rapport à ladite partie fixe (3; 101) entre une position de fermeture, dans laquelle le récipient porte-argent ouvrable (1; 100) est fermé et la pluralité de sièges (13; 108) est complètement enfermée par le récipient porte-argent ouvrable (1; 100), et une position d'ouverture, dans laquelle le récipient porte-argent ouvrable (1; 100) est ouvert et la pluralité de sièges (13; 108) est exposée à l'extérieur du récipient porte-argent ouvrable (1; 100), à savoir accessible depuis l'extérieur du récipient porte-argent ouvrable (1; 100);
- au moins un émetteur de rayons UVC (7; 104), ou au moins une lampe à ozone, raccordé à ladite partie fixe (3; 101) ou à ladite partie mobile (5; 105) et disposé dans une position lui permettant d'émettre, en cours d'utilisation, au moins un faisceau vers les pièces de monnaie et/ou jetons et/ou billets de banque et/ou chèques et/ou bons d'achat et/ou chèques-repas disposés dans la pluralité de sièges (13; 108) lorsque ladite partie mobile (5; 105) est dans ladite position de fermeture, de manière à réduire ou annuler la charge de virus et/ou bactéries et/ou spores et/ou moisissures et/ou autres pathogènes éventuellement présents dans lesdites pièces de monnaie et/ou lesdits jetons et/ou lesdits billets de banque et/ou lesdits chèques et/ou lesdits bons d'achat et/ou lesdits chèques-repas; et
- au moins un dispositif de circulation d'air (15; 111) destiné à faire circuler de l'air à l'intérieur du récipient porte-argent ouvrable (1; 100);
où l'utilisation est **caractérisée en ce que** les pièces de monnaie et/ou jetons et/ou billets de banque et/ou chèques et/ou bons d'achat et/ou chèques-repas sont disposés verticalement dans les sièges (13; 108), séparés les uns des autres.

2. Utilisation du récipient porte-argent ouvrable (1; 100) selon la revendication 1, **caractérisée en ce que** ledit au moins un émetteur de rayons UVC (7; 104), ou ladite au moins une lampe à ozone, est disposé au-dessus de la pluralité de sièges (13; 108) lorsque ladite partie mobile (5; 105) est dans la position de fermeture.

3. Utilisation du récipient porte-argent ouvrable (1) selon l'une quelconque des revendications précédentes, **caractérisée en ce que** ladite partie fixe (3) présente une partie creuse (4) configurée pour loger la pluralité de sièges (13).

4. Utilisation du récipient porte-argent ouvrable (1) selon la revendication 3, **caractérisée en ce que** ladite partie mobile (5) est reliée de manière rotative à ladite partie fixe (3).

5. Utilisation du récipient porte-argent ouvrable (1) selon la revendication 3 ou 4, **caractérisée en ce que** ledit au moins un émetteur de rayons UVC (7), ou ladite au moins une lampe à ozone, est raccordé à ladite partie mobile (5).

6. Utilisation du récipient porte-argent ouvrable (1) selon l'une quelconque des revendications de 3 à 5, **caractérisée en ce que** ledit au moins un dispositif de circulation d'air (15) est relié à ladite partie fixe (3) au niveau d'une partie de fond de la partie creuse (4).

7. Utilisation du récipient porte-argent ouvrable (100) selon la revendication 1 ou 2, **caractérisée en ce que** ladite partie mobile (105) présente une partie creuse configurée pour loger la pluralité de sièges (108).

8. Utilisation du récipient porte-argent ouvrable (100) selon la revendication 7, **caractérisée en ce que** ladite partie mobile (105) est reliée à ladite partie fixe (101) de manière coulissante.

9. Utilisation du récipient porte-argent ouvrable (100) selon la revendication 7 ou 8, **caractérisée en ce que** ledit au moins un émetteur de rayons UVC (104), ou ladite au moins une lampe à ozone, est raccordé à ladite partie fixe (101).

10. Utilisation du récipient porte-argent ouvrable (100) selon l'une quelconque des revendications de 7 à 9, **caractérisée**
**en ce que**, dans ladite partie fixe (101), un compartiment (102) destiné à loger ladite partie mobile (105) et une ouverture (103) communiquant avec le compartiment (102) sont définis; et
**en ce que** ledit au moins un dispositif de circulation d'air (111) est relié à ladite partie fixe (101) au niveau d'une zone opposée à ladite ouverture (103).

11. Utilisation du récipient porte-argent ouvrable (1; 100) selon l'une quelconque des revendications précédentes, **caractérisée en ce que** la pluralité de sièges (13; 108) est amovible.

12. Utilisation du récipient porte-argent ouvrable (1; 100) selon l'une quelconque des revendications précédentes, **caractérisée en ce qu'**au moins un siège de ladite pluralité de sièges (13; 108) est réalisé dans un matériau qui est au moins partiellement transparent audit au moins un rayon.

13. Utilisation du récipient porte-argent ouvrable (1; 100) selon l'une quelconque des revendications précédentes, **caractérisée en ce qu'**il comprend un dispositif de sécurité (16; 110) opérationnellement relié audit au moins un émetteur de rayons UVC (104), ou à ladite au moins une lampe à ozone, et configuré de manière à désactiver automatiquement ledit au moins un émetteur de rayons UVC (104) ou ladite au moins une lampe à ozone lorsque ladite partie mobile (5; 105) se déplace de la position de fermeture à la position d'ouverture.

14. Utilisation du récipient porte-argent ouvrable (1; 100) selon l'une quelconque des revendications précédentes, **caractérisée en ce qu'**il comprend un élément de commande pour permettre à une personne de commander volontairement la désactivation dudit au moins un émetteur de rayons UVC (104) ou ladite au moins une lampe à ozone.

15. Utilisation du récipient porte-argent ouvrable (1; 100) selon l'une quelconque des revendications précédentes, **caractérisée en ce qu'**il comprend au moins une surface qui réfléchit ledit au moins un rayon et est définie sur un côté de ladite partie mobile (5; 105) ou de ladite partie fixe (3; 101) orientée vers la pluralité de sièges (13; 108) lorsque ladite partie mobile (5; 105) est dans la position de fermeture.

16. Utilisation du récipient porte-argent ouvrable (1; 100) selon l'une quelconque des revendications précédentes, **caractérisée en ce qu'**au moins un siège de ladite pluralité de sièges (13; 108) est réalisé en verre de quartz ou en polydiméthylsiloxane (PDMS) ou en éthylène propylène fluoré (FEP).
